# EUROPEAN PATENT APPLICATION

(11) **EP 4 814 113 A1**
(43) Date of publication of application: **30.09.2026**
(21) Application number: 24938434.8
(22) Date of filing: 26.07.2024
(51) Int. Cl.: C12N 9/02, C12P 17/02

(54) **SYNTHESIS METHOD FOR CHIRAL EPOXIDE CATALYZED BY STYRENE MONOOXYGENASE**

(30) Priority: 14.05.2024 CN 202410594631
(71) Applicant: Tianjin Asymchem Biotechnology Co., Ltd., Tianjin 300457 (CN); Asymchem Laboratories (Tianjin) Co., Ltd., Tianjin 300457 (CN)
(72) Inventor: HONG, Hao, Morrisville, North Carolina 27560 (US); ZHANG, Na, Tianjin 300457 (CN); JIAO, Xuecheng, Tianjin 300457 (CN); MA, Yulei, Tianjin 300457 (CN); CHEN, Kun, Tianjin 300457 (CN); LI, Hongxuan, Tianjin 300457 (CN); WANG, Yuyin, Tianjin 300457 (CN); MA, Tianjiao, Tianjin 300457 (CN)
(74) Representative: Office Freylinger
(86) International application number: PCT/CN2024/107940
(87) International publication number: WO 2025/236419

(57) **Abstract**

Provided is a method for synthesizing chiral epoxide catalyzed by styrene monooxygenase. The synthesis method includes: performing an enzyme catalysis reaction on a raw material system including oxidase, an olefin compound, reductase, dehydrogenase, a hydrogen donor, FAD, and NAD⁺, so as to obtain chiral epoxide, where R₂ and R₃ are each independently selected from substituents such as H, substituted or unsubstituted C₁-C₆ alkyl, substituted or unsubstituted C₁-C₆ heteroalkyl, a substituted or unsubstituted C₂-C₆ ester group, etc., and R₁ is selected from substituents such as halogen, hydroxy, carboxyl, acetoxy, substituted or unsubstituted C₁-C₆ alkyl, etc. The raw material system is used for performing the enzyme catalysis reaction, and the oxidase therein is used to obtain the chiral epoxide. The method for synthesizing chiral epoxide catalyzed by oxidase has the advantages of a short synthesis route, a wide range of substrate types, high selectivity, mild reaction conditions, less three wastes, and low costs.

## Description

This application is based upon and claims priority to Chinese Patent Application No. 202410594631.4 filed on May 14, 2024, the disclosure of which is hereby incorporated by reference in its entirety.

### Technical Field

The present disclosure belongs to the technical field of methods for synthesizing chiral epoxide, and specifically to method for synthesizing chiral epoxide catalyzed by styrene monooxygenase.

### Background

Large-steric-hindrance chiral epoxide (referring to a class of compounds in which at least one of the groups on either side of a ternary ether structure is larger than methyl in the present disclosure) is a very important class of chiral synthetic fragments in organic synthesis, and has significant application values in the pharmaceutical, pesticide, fragrance, and fine chemicals industries. At present, precious metal catalysts and crystalline titanosilicate catalysts with an MFI structure are primarily used as dual-catalysts to catalyze epoxide of an olefin substrate. However, chemical catalysis is harsh in reaction conditions, low in selectivity, high in by-product level, high in heavy metal catalyst used, and unfriendly to environment. Currently, there have been few reports on the synthesis of the large-steric-hindrance chiral epoxide, and satisfactory results have not been achieved. (Jaan Pesti*, Chien-Kuang Chen*, Lori Spangler, Albert J. DelMonte, Serge Benoit, Derek Berglund, Jeffrey Bien, Paul Brodfuehrer, Yeung Chan, Elisabeth Corbett, Carrie Costello, Paul DeMena, Robert P. Discordia, Wendel Doubleday, Zhinong Gao, Stephane Gingras, John Grosso, Oscar Haas, David Kacsur, Chiajen Lai, Simon Leung, Melanie Miller, Jale Muslehiddinoglu, Nina Nguyen, Jun Qiu, Martina Olzog, Emily Reiff, Dominique Thoraval, Michael Totleben, Dale Vanyo, Purushotham Vemishetti, John Wasylak, and Chenkou Wei. The Process Development of Ravuconazole: An Efficient Multikilogram Scale Preparation of an Antifungal Agent. Organic Process Research & Development. 2009, 13(4):716-728) disclosed a method for synthesizing a large-steric-hindrance chiral epoxide intermediate. However, the method is relatively long in synthetic route and needs to perform protection and deprotection on an active group, leading to technical problems of harsh reaction conditions, poor selectivity, and complex process.

Therefore, methods for synthesizing large-steric-hindrance chiral epoxide in the related art are high in cost and complex in process, such that a new synthesis method is urgently provided to replace current traditional methods.

### Summary

A main objective of the present disclosure is to provide a method for synthesizing chiral epoxide catalyzed by styrene monooxygenase, so as to solve the problems of methods for synthesizing chiral epoxide in the related art of being long in process route, complex in synthesis process, poor in selectivity, high in synthesis cost, and large in pollution.

In order to implement the above objective, an aspect of the present disclosure provides a method for synthesizing chiral epoxide catalyzed by styrene monooxygenase. The synthesis method includes: an enzyme catalysis reaction is performed on a raw material system including oxidase, an olefin compound, reductase, dehydrogenase, a hydrogen donor, FAD, and NAD⁺, so as to obtain chiral epoxide, where structural formulas of the olefin compound and chiral epoxide respectively are as follows.

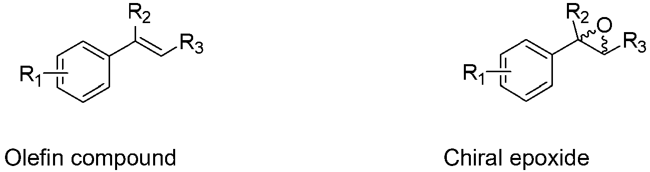

R₂ and R₃ are each independently selected from any one of H, substituted or unsubstituted C₁-C₆ alkyl, substituted or unsubstituted C₁-C₆ heteroalkyl, a substituted or unsubstituted C₂-C₆ ester group, substituted or unsubstituted C₁-C₄ acylamino, substituted or unsubstituted C₃-C₆ cycloalkyl, substituted or unsubstituted C₃-C₆ cycloenyl, or substituted or unsubstituted C₆-C₁₂ aryl; and R₁ is selected from any one of halogen, hydroxy, carboxyl, acetoxy, substituted or unsubstituted C₁-C₆ alkyl, or substituted or unsubstituted C₁-C₆ heteroalkyl.

Further, a heteroatom on the heteroalkyl is selected from any one or more of N, O, and S; and R₂ and R₃ are each independently selected from any one of H, substituted or unsubstituted C₁-C₄ alkyl, substituted or unsubstituted C₁-C₄ alkoxy, a substituted or unsubstituted C₂-C₄ ester group, substituted or unsubstituted C₁-C₂ acylamino, substituted or unsubstituted C₃-C₅ cycloalkyl, substituted or unsubstituted C₃-C₅ cycloenyl, or substituted or unsubstituted C₆-C₁₀ aryl.

Further, R₂ and R₃ are each independently selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl; and when substituents are present on R₂ and R₃, the substituents are selected from any one or more of hydroxy, carboxyl, methyl, ethyl, nitro, methylamino, acetoxy, and five-membered ring heteroaryl or six-membered ring heteroaryl.

Further, R₂ and R₃ are each independently selected from any one of H, methyl, ethyl, phenyl, 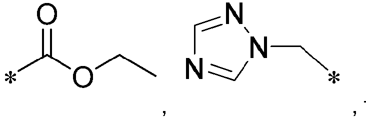 formamido, hydroxymethyl, or cyclopentadienyl.

Further, the heteroatom on the heteroalkyl is selected from any one or more of N, O, and S; R₁ is selected from any one of halogen, hydroxy, carboxyl, substituted or unsubstituted C₁-C₄ alkyl, or substituted or unsubstituted C₁-C₄ alkoxy; and when a substituent is present on R₁, the substituent is selected from any one or more of hydroxy, carboxyl, methyl, ethyl, nitro, methylamino, and acetoxy.

Further, R₁ is selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl.

Further, R₁ is selected from any one of F, Cl, hydroxyl, methyl, ethyl, methoxy, hydroxymethyl, 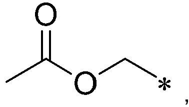 acetoxy.

Further, the olefin compound is selected from any one or more of

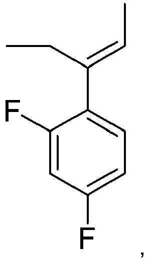

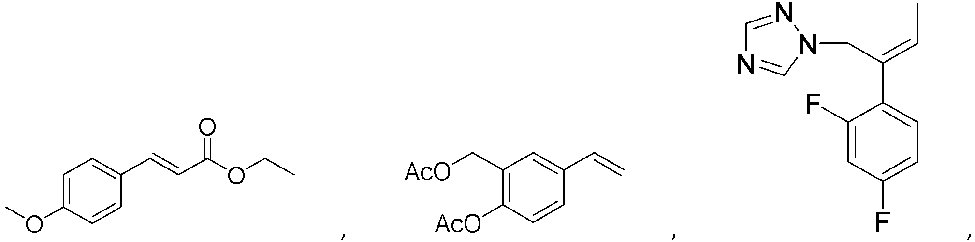

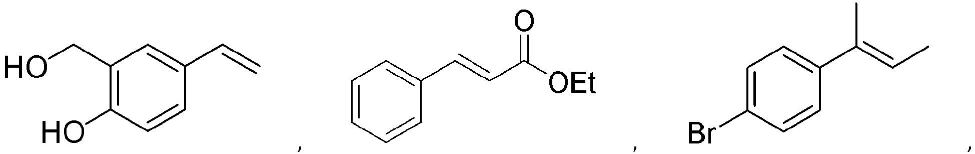

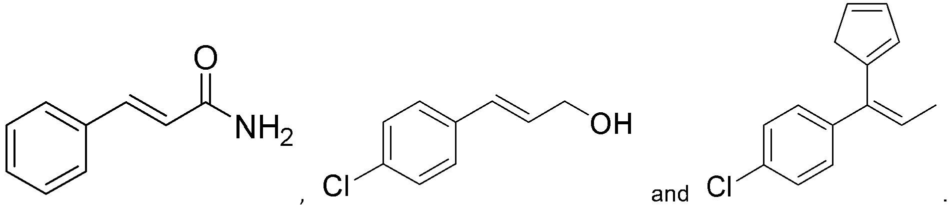

Further, the oxidase is selected from any one or more of oxidase with sequence being derived from *Marinobacterium litorale* DSM 23545 and NCBI serial number being WP_027855270.1, oxidase with sequence being derived from *Sphingopyxis fribergensis* and NCBI serial number being AJA07151.1, oxidase with sequence being derived from *Streptomyces exfoliatus* and NCBI serial number being WP_137992763.1, oxidase with sequence being derived from *Bradyrhizobium* sp. ORS 375 and NCBI serial number being WP_009031806.1, oxidase with sequence being derived from *Amycolatopsis albispora* and NCBI serial number being WP_113694022.1, oxidase with sequence being derived from *Celeribacter baekdonensis* and NCBI serial number being WP_107722930.1, oxidase with sequence being derived from *Rhodococcus* sp. ST-5 and NCBI serial number being BAL04132.1, oxidase with sequence being derived from *Nocardia farcinica* and NCBI serial number being MBF6535966.1, oxidase with sequence being derived from *Pseudomonas aeruginosa* and NCBI serial number being WP_121207564.1, oxidase with sequence being derived from *Pseudomonas oryzae* and NCBI serial number being WP_090348206.1, oxidase with sequence being derived from *Pseudomonas* sp. SXM-1 and NCBI serial number being WP_134326109.1, oxidase with sequence being derived from *Pseudonocardia* sp. SID8383 and NCBI serial number being WP_161151635.1, oxidase with sequence being derived from *Streptomyces* sp. WAC01280 and NCBI serial number being WP_125743710.1, oxidase with sequence being derived from *Pseudomonas putida* S12 and NCBI serial number being AJA17113.1, and oxidase with sequence being derived from *Variovorax paradoxus* EPS and NCBI serial number being ADU39062.1.

Further, the oxidase is selected from any one or more of the oxidase with the sequence being derived from *Sphingopyxis fribergensis* and NCBI serial number being AJA07151.1, the oxidase with the sequence being derived from *Bradyrhizobium* sp. ORS 375 and NCBI serial number being WP_009031806.1, and the oxidase with the sequence being derived from *Rhodococcus* sp. ST-5 and NCBI serial number being BAL04132.1, the oxidase with the sequence being derived from *Pseudomonas aeruginosa* and NCBI serial number being WP_121207564.1, and the oxidase with the sequence being derived from *Pseudomonas oryzae* and NCBI serial number being WP_090348206.1.

Further, a temperature for the enzyme catalysis reaction is 20-40°C, and/or a time for the enzyme catalysis reaction is 16-72 h.

Further, a mass ratio of the oxidase in the raw material system to the raw material system is 10-50:1; and/or the mass of the oxidase is 20-100 mg; and/or a mass ratio of the reductase to the raw material system is 1-10:1; and/or the mass of the reductase is 2-20 mg; and/or a mass ratio of the hydrogen donor to the raw material system is 1-10:1; a mass ratio of the total mass of the FAD and the NAD⁺ to the raw material system is 10-1000:1; and/or the reductase is reductase StyB that is able to regenerate FADH₂, and/or the dehydrogenase and the hydrogen donor respectively are Glucose Dehydrogenase (GDH) and glucose; and/or the dehydrogenase and the hydrogen donor respectively are alcohol dehydrogenase and isopropanol; and/or a mass concentration of the olefin compound is 0.5-2 mg/mL; and/or a source of the reductase StyB is selected from any one or more of *Pseudomonas* sp. LQ26, *Bradyrhizobium* sp. ORS 375, Pseudomonas putida, Pseudomonas fluorescens, Pseudomonas sp. Y2, *Rhodococcus* sp. ST-5, and *Rhodococcus* sp. ST-10; and/or the raw material system includes a buffer solution, the buffer solution is selected from any one or more of a phosphate buffer solution, a Tris-HCl buffer solution, and a boric acid buffer solution, and/or a pH value of the buffer solution is 7.5-10.

By using the technical solutions of the present disclosure, although the steric hindrance at both ends of an ethylene double bond in the olefin compound having the above structure is relatively large, the present disclosure uses the above raw material system to perform the enzyme catalysis reaction and use the oxidase therein to obtain the chiral epoxide. Compared to a traditional metal catalyst synthesis method, the method for synthesizing chiral epoxide catalyzed by oxidase has the advantages of a short synthesis route, a wide range of substrate types, high selectivity, mild reaction conditions, less three wastes, and low costs.

### Detailed Description of the Embodiments

It is to be noted that the embodiments in the present disclosure and the features in the embodiments may be combined with one another without conflict. The present disclosure will be described below in detail with reference to the embodiments.

As analyzed in the Background of the present disclosure, methods for synthesizing chiral epoxide in the related art have the problems of being long in process route, complex in synthesis process, poor in selectivity, high in synthesis cost, and large in pollution. In order to solve the problems, the present disclosure provides a method for synthesizing chiral epoxide catalyzed by styrene monooxygenase.

In a typical implementation of the present disclosure, a method for synthesizing chiral epoxide catalyzed by styrene monooxygenase is provided. The synthesis method includes: an enzyme catalysis reaction is performed on a raw material system including oxidase, an olefin compound, reductase, dehydrogenase, a hydrogen donor, FAD, and NAD⁺, so as to obtain chiral epoxide, where structural formulas of the olefin compound and chiral epoxide respectively are as follows:

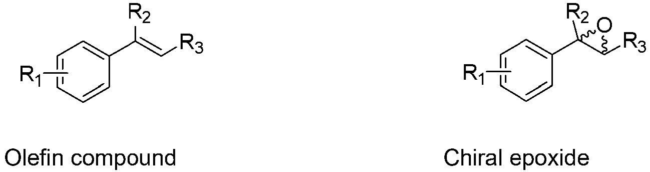

R₂ and R₃ are each independently selected from any one of H, substituted or unsubstituted C₁-C₆ alkyl, substituted or unsubstituted C₁-C₆ heteroalkyl, a substituted or unsubstituted C₂-C₆ ester group, substituted or unsubstituted C₁-C₄ acylamino, substituted or unsubstituted C₃-C₆ cycloalkyl, substituted or unsubstituted C₃-C₆ cycloenyl, or substituted or unsubstituted C₆-C₁₂ aryl; and R₁ is selected from any one of halogen, hydroxy, carboxyl, acetoxy, substituted or unsubstituted C₁-C₆ alkyl, or substituted or unsubstituted C₁-C₆ heteroalkyl.

Although the steric hindrance at both ends of an ethylene double bond in the olefin compound having the above structure is relatively large, the present disclosure uses the above raw material system to perform the enzyme catalysis reaction and use the oxidase therein to obtain the chiral epoxide. Compared to a traditional metal catalyst synthesis method, the method for synthesizing chiral epoxide catalyzed by oxidase has the advantages of a short synthesis route, a wide range of substrate types, high selectivity, mild reaction conditions, less three wastes, and low costs.

In an embodiment of the present disclosure, a heteroatom on the heteroalkyl is preferably selected from any one or more of N, O, and S; and R₂ and R₃ are each independently selected from any one of H, substituted or unsubstituted C₁-C₄ alkyl, substituted or unsubstituted C₁-C₄ alkoxy, a substituted or unsubstituted C₂-C₄ ester group, substituted or unsubstituted C₁-C₂ acylamino, substituted or unsubstituted C₃-C₅ cycloalkyl, substituted or unsubstituted C₃-C₅ cycloenyl, or substituted or unsubstituted C₆-C₁₀ aryl, such that the selectivity of the olefin compound is further enriched.

In an embodiment of the present disclosure, R₂ and R₃ are each preferably independently selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl; and when substituents are present on R₂ and R₃, the substituents are selected from any one or more of hydroxy, carboxyl, methyl, ethyl, nitro, methylamino, acetoxy, and five-membered ring heteroaryl or six-membered ring heteroaryl. Preferably, the five-membered ring heteroaryl is nitrogen-containing heterocyclic aryl, and the nitrogen-containing heterocyclic aryl is preferably 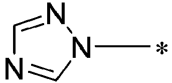 such that the selectivity of the olefin compound is further enriched.

In an embodiment of the present disclosure, R₂ and R₃ are each preferably independently selected from any one of H, methyl, ethyl, phenyl, 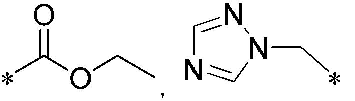 formamido, hydroxymethyl, or cyclopentadienyl, such that the selectivity of the olefin compound is further enriched.

In an embodiment of the present disclosure, the heteroatom on the heteroalkyl is preferably selected from any one or more of N, O, and S; R₁ is selected from any one of halogen, hydroxy, carboxyl, substituted or unsubstituted C₁-C₄ alkyl, or substituted or unsubstituted C₁-C₄ alkoxy; and when a substituent is present on R₁, the substituent is selected from any one or more of hydroxy, carboxyl, methyl, ethyl, nitro, methylamino, and acetoxy, such that the selectivity of the olefin compound is further enriched.

In an embodiment of the present disclosure, R₁ is preferably selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl, such that the selectivity of the olefin compound is further enriched.

In an embodiment of the present disclosure, R₁ is preferably selected from any one of F, Cl, hydroxyl, methyl, ethyl, methoxy, hydroxymethyl, 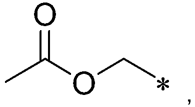 acetoxy, such that the selectivity of the olefin compound is further enriched.

In an embodiment of the present disclosure, the olefin compound is selected from any one or more of 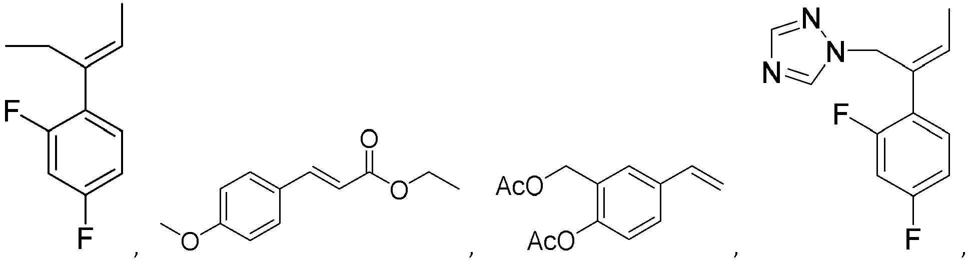 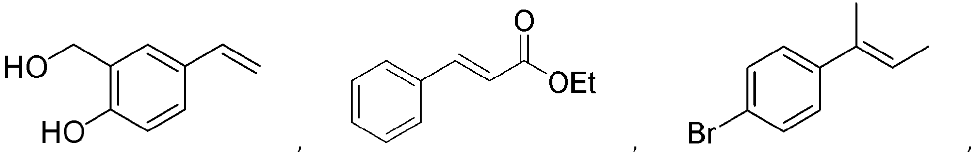 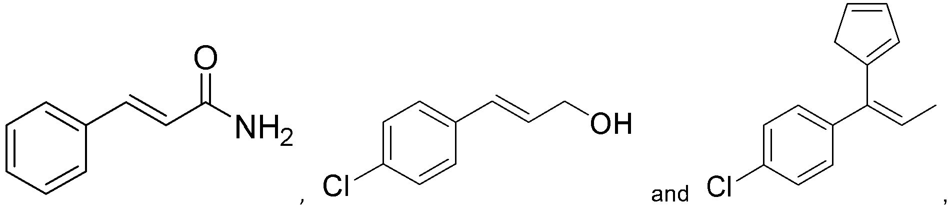 such that the types of the chiral epoxide are further enriched.

In an embodiment of the present disclosure, the oxidase is selected from any one or more of oxidase with sequence being derived from *Marinobacterium litorale* DSM 23545 and NCBI serial number being WP_027855270.1, oxidase with sequence being derived from *Sphingopyxis fribergensis* and NCBI serial number being AJA07151.1, oxidase with sequence being derived from *Streptomyces exfoliatus* and NCBI serial number being WP_137992763.1, oxidase with sequence being derived from *Bradyrhizobium* sp. ORS 375 and NCBI serial number being WP_009031806.1, oxidase with sequence being derived from *Amycolatopsis albispora* and NCBI serial number being WP_113694022.1, oxidase with sequence being derived from *Celeribacter baekdonensis* and NCBI serial number being WP_107722930.1, oxidase with sequence being derived from *Rhodococcus* sp. ST-5 and NCBI serial number being BAL04132.1, oxidase with sequence being derived from *Nocardia farcinica* and NCBI serial number being MBF6535966.1, oxidase with sequence being derived from *Pseudomonas aeruginosa* and NCBI serial number being WP_121207564.1, oxidase with sequence being derived from *Pseudomonas oryzae* and NCBI serial number being WP_090348206.1, oxidase with sequence being derived from *Pseudomonas* sp. SXM-1 and NCBI serial number being WP_134326109.1, oxidase with sequence being derived from *Pseudonocardia* sp. SID8383 and NCBI serial number being WP_161151635.1, oxidase with sequence being derived from *Streptomyces* sp. WAC01280 and NCBI serial number being WP_125743710.1, oxidase with sequence being derived from *Pseudomonas putida* S12 and NCBI serial number being AJA17113.1, and oxidase with sequence being derived from *Variovorax paradoxus* EPS and NCBI serial number being ADU39062.1.

Preferably, the oxidase facilitates conversion of the olefin compound with large steric hindrance in the present disclosure into the chiral epoxide.

Further, the oxidase is preferably selected from any one or more of the oxidase with the sequence being derived from *Sphingopyxis fribergensis* and NCBI serial number being AJA07151.1, the oxidase with the sequence being derived from *Bradyrhizobium* sp. ORS 375 and NCBI serial number being WP_009031806.1, and the oxidase with the sequence being derived from *Rhodococcus* sp. ST-5 and NCBI serial number being BAL04132.1, the oxidase with the sequence being derived from *Pseudomonas aeruginosa* and NCBI serial number being WP_121207564.1, and the oxidase with the sequence being derived from *Pseudomonas oryzae* and NCBI serial number being WP_090348206.1, such that the efficiency of converting the olefin compound into the chiral epoxide is improved.

In an embodiment of the present disclosure, a temperature for the enzyme catalysis reaction is preferably 20-40 °C, preferably 25-35 °C, and/or a time for the enzyme catalysis reaction is 16-72 h, such that the efficiency of converting the olefin compound into the chiral epoxide is improved.

In an embodiment of the present disclosure, a mass ratio of the oxidase in the raw material system to the raw material system is 10-50:1; and/or the mass of the oxidase is 20-100 mg; and/or a mass ratio of the reductase to the raw material system is 1-10:1; and/or the mass of the reductase is 2-20 mg; and/or a mass ratio of the hydrogen donor to the raw material system is 1-10:1; a mass ratio of the total mass of the FAD and the NAD⁺ to the raw material system is 10-1000:1; and/or the reductase is reductase StyB that is able to regenerate FADH₂, and/or the dehydrogenase and the hydrogen donor respectively are Glucose Dehydrogenase and glucose; and/or the dehydrogenase and the hydrogen donor respectively are alcohol dehydrogenase and isopropanol; and/or a mass concentration of the olefin compound is 0.5-2 mg/mL; and/or a source of the reductase StyB is selected from any one or more of *Pseudomonas* sp. LQ26, *Bradyrhizobium* sp. ORS 375, *Pseudomonas putida, Pseudomonas fluorescens, Pseudomonas* sp. Y2, *Rhodococcus* sp. ST-5, and *Rhodococcus* sp. ST-10; and/or the raw material system includes a buffer solution, the buffer solution is selected from any one or more of a phosphate buffer solution, a Tris-HCl buffer solution, and a boric acid buffer solution, and/or a pH value of the buffer solution is 7.5-10.

The above preferred conditions further optimize the reaction conditions for converting the olefin compound into the chiral epoxide. Under the above reaction conditions, the efficiency of converting the olefin compound into the chiral epoxide is further improved.

The beneficial effects of the present disclosure were further described below with reference to the embodiments.

The olefin compounds involved in embodiments below are as follows.

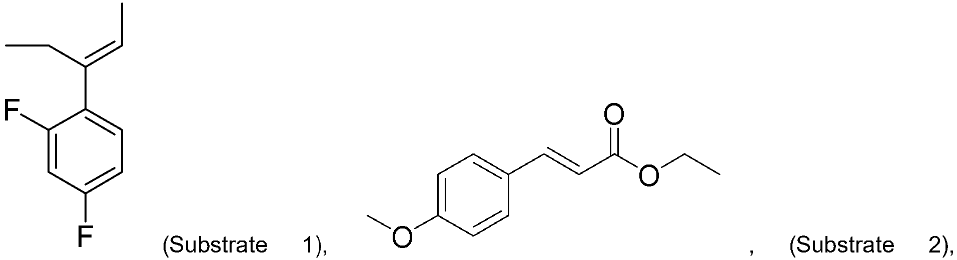

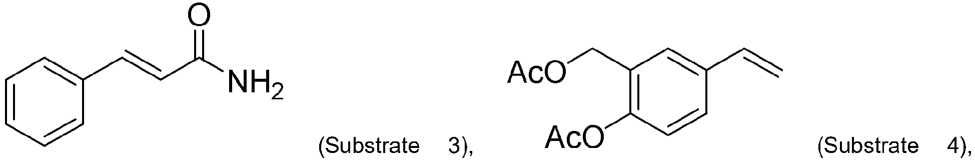

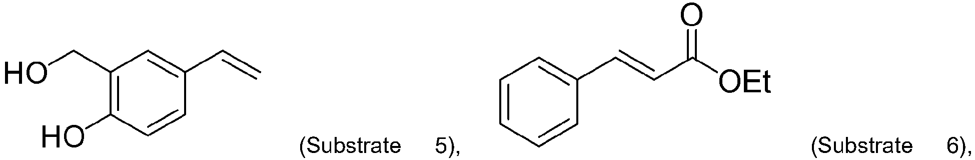

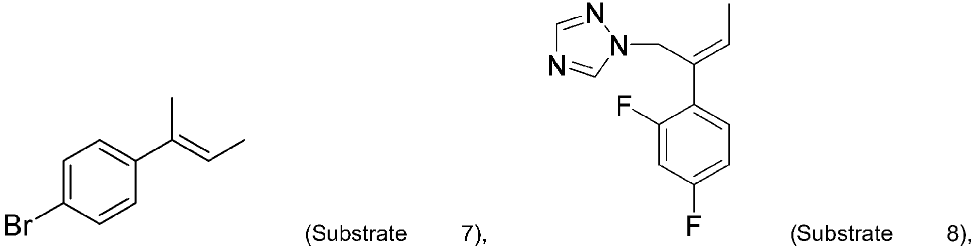

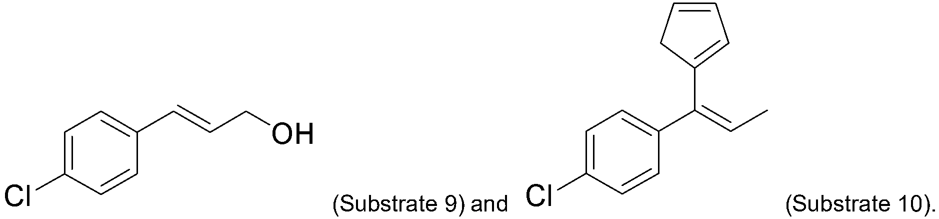

### Embodiment 1

In order to solve the problems of being long in existing technical route, harsh in reaction condition, poor in selectivity, complex in process, and high in cost, the inventor tried to use a biological enzyme method to perform synthesis of large-steric-hindrance chiral epoxide. However, no styrene monooxygenase capable of synthesizing large-steric-hindrance chiral epoxide has been reported in the related art. In order to improve this situation, the inventor performed extensive screening on existing enzyme libraries of Asymchem Labs., hundreds of redox enzymes including the styrene monooxygenase were screened, and most enzymes showed no activity (no product was detected by HPLC), but a few enzymes demonstrated some catalytic activity in converting a large-steric-hindrance substrate into chiral epoxide. Details were showed as follows.

Screening and verification of a large-steric-hindrance olefin substrate were performed on oxidase from different species.

This embodiment selected hundreds of oxidase in the enzyme libraries of Asymchem Labs. to perform enzyme screening of an olefin substrate 1. A screening system was as follows: a 1 mL reaction system included 1 mg of the substrate, 50 mg of enzyme powder of styrene monooxygenase StyA, 10 mg of enzyme powder of reductase StyB, 5 mg of enzyme powder of Glucose Dehydrogenase (GDH), 10 mg of glucose, 100 µM of FAD, 20 mM of NAD⁺, and 100 mM of a phosphate buffer solution with a pH value being 7.0, and the reaction was performed for 2 h at 30 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC.

Results showed that, generation of enzyme products was not detected in most enzyme libraries, but a few enzyme products were generated, with a conversion rate reaching up to approximately 30%. These enzymes included the following sequences (shown in Table 1).

**Table 1**

| Oxidase number | Sequence source | NCBI serial number | Conversion rate |
|---|---|---|---|
| 1 | *Marinobacterium litorale* DSM 23545 | WP_027855270.1 | ** |
| 2 | *Sphingopyxis fribergensis* | AJA07151.1 | **** |
| 3 | *Streptomyces exfoliatus* | WP_137992763.1 | * |
| 4 | *Bradyrhizobium* sp. ORS 375 | WP_009031806.1 | *** |
| 5 | *Amycolatopsis albispora* | WP_113694022.1 | * |
| 6 | *Celeribacter baekdonensis* | WP_107722930.1 | ** |
| 7 | *Rhodococcus* sp. ST-5 | BAL04132.1 | *** |
| 8 | *Nocardia farcinica* | MBF6535966.1 | * |
| 9 | *Pseudomonas aeruginosa* | WP_121207564.1 | *** |
| 10 | *Pseudomonas oryzae* | WP_090348206.1 | *** |
| 11 | *Pseudomonas* sp. SXM-1 | WP_134326109.1 | * |
| 12 | *Pseudonocardia* sp. SID8383 | WP_161151635.1 | ** |
| 13 | *Streptomyces* sp. WAC01280 | WP_125743710.1 | ** |
| 14 | *Pseudomonas putida* S12 | AJA17113.1 | * |
| 15 | *Variovorax paradoxus* EPS | ADU39062.1 | * |

| | | | |
|---|---|---|---|
| Note: in Table 1 above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | | | |

### Embodiment 2

Optimization of reaction conditions:
StyA that had good activity to an olefin substrate 1 and was derived from *Sphingopyxis fribergensis* was selected to perform reaction optimization, and single-factor optimization experiments with different temperatures, pH, substrate concentrations, StyB, GDH, FAD, NAD⁺, and glucose were respectively set.

Reaction condition 1: a 1 mL reaction system included 1 mg of the olefin substrate 1, 50 mg of enzyme powder of styrene monooxygenase StyA, 10 mg of enzyme powder of reductase StyB, 5 mg of enzyme powder of Glucose Dehydrogenase (GDH), 10 mg of glucose, 100 µM of FAD, 20 mM of NAD⁺, and 100 mM of a phosphate buffer solution with a pH value being 7.0, and the reaction was performed for 16 h at 20 °C, 25 °C, 30 °C, 35 °C, and 40 °C, respectively. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 2.

**Table 2**

| Temperature (°C) | Conversion rate |
|---|---|
| 20 | - |
| 25 | - |
| 30 | -+ |
| 35 | + |
| 40 | - |

| | |
|---|---|
| Note: in Table 2 above, - indicated a decrease in conversion rate; + indicated an increase in conversion rate; and -+ indicated no change in conversion rate. | |

According to the results in Table 2, next optimization was performed by selecting 35 °C.

Reaction condition 2: a 1 mL reaction system included 1 mg of the olefin substrate 1, 50 mg of enzyme powder of styrene monooxygenase StyA, 10 mg of enzyme powder of reductase StyB, 5 mg of enzyme powder of Glucose Dehydrogenase (GDH), 10 mg of glucose, 100 µM of FAD, 20 mM of NAD⁺, and 100 mM of a phosphate buffer solution with different pH values, and the reaction was performed for 16 h at 30 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 3.

**Table 3**

| pH | Conversion rate |
|---|---|
| 6.5 | - |
| 7.0 | -+ |
| 7.5 | + |
| 8.0 | - |
| 8.5 | - |

| | |
|---|---|
| Note: in the table above, - indicated a decrease in conversion rate; + indicated an increase in conversion rate; and -+ indicated no change in conversion rate. | |

According to the results in Table 3, next optimization was performed by selecting the pH value as 7.5.

Reaction condition 3: a 1 mL reaction system included different amounts of the olefin substrate 1, 50 mg of enzyme powder of styrene monooxygenase StyA, 10 mg of enzyme powder of reductase StyB, 5 mg of enzyme powder of Glucose Dehydrogenase (GDH), 10 mg of glucose, 100 µM of FAD, 20 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 4.

**Table 4**

| Concentration of olefin substrate (mg/mL) | Conversion rate |
|---|---|
| 0.5 | + |
| 1 | -+ |
| 1.5 | + |
| 2 | + |
| 2.5 | - |
| 3 | - |
| 3.5 | - |
| 4 | - |

| | |
|---|---|
| Note: in the table above, - indicated a decrease in conversion rate; + indicated an increase in conversion rate; and -+ indicated no change in conversion rate. | |

According to the results in Table 4, next optimization was performed by selecting the concentration of the olefin substrate as 2 mg/mL.

Reaction condition 4: a 1 mL reaction system included 2 mg of the olefin substrate 1, 50 mg of enzyme powder of styrene monooxygenase StyA, 10 mg of enzyme powder of reductase StyB from different sources, 5 mg of enzyme powder of Glucose Dehydrogenase (GDH), 10 mg of glucose, 100 µM of FAD, 20 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 5.

**Table 5**

| StyB source | Conversion rate |
|---|---|
| *Pseudomonas* sp. LQ26 | + |
| *Bradyrhizobium* sp. ORS 375 | -+ |
| *Pseudomonas putida* | + |
| *Pseudomonas fluorescens* | -+ |
| *Pseudomonas* sp. Y2 | + |
| *Rhodococcus* sp. ST-5 | + |
| *Rhodococcus* sp. ST-10 | -+ |

| | |
|---|---|
| Note: in the table above, - indicated a decrease in conversion rate; + indicated an increase in conversion rate; and -+ indicated no change in conversion rate. | |

According to the results in Table 4, next optimization was performed by selecting the StyB derived from *Pseudomonas putida.*

Reaction condition 5: a 1 mL reaction system included 2 mg of the olefin substrate 1, 50 mg of enzyme powder of styrene monooxygenase StyA, 10 mg of enzyme powder of reductase StyB, different amounts of enzyme powder of Glucose Dehydrogenase (GDH), 10 mg of glucose, 100 µM of FAD, 20 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 6.

**Table 6**

| GDH (mg) | Conversion rate |
|---|---|
| 1 | - |
| 2.5 | - |
| 5 | -+ |
| 7.5 | + |
| 10 | + |

| | |
|---|---|
| Note: in the table above, - indicated a decrease in conversion rate; + indicated an increase in conversion rate; and -+ indicated no change in conversion rate. | |

According to the results in Table 6, next optimization was performed by selecting 7.5 mg of the GDH.

Reaction condition 6: a 1 mL reaction system included 2 mg of the olefin substrate 1, 50 mg of enzyme powder of styrene monooxygenase StyA, 10 mg of enzyme powder of reductase StyB, 7.5 mg of enzyme powder of Glucose Dehydrogenase (GDH), 10 mg of glucose, different amounts of FAD, 20 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 7.

**Table 7**

| FAD (µM) | Conversion rate |
|---|---|
| 20 | - |
| 25 | -+ |
| 50 | -+ |
| 100 | -+ |
| 125 | - |
| 150 | - |

| | |
|---|---|
| Note: in the table above, - indicated a decrease in conversion rate; + indicated an increase in conversion rate; and -+ indicated no change in conversion rate. | |

According to the results in Table 7, next optimization was performed by selecting 25 µM of the FAD.

Reaction condition 7: a 1 mL reaction system included 2 mg of the olefin substrate 1, 50 mg of enzyme powder of styrene monooxygenase StyA, 10 mg of enzyme powder of reductase StyB from different sources, 7.5 mg of enzyme powder of Glucose Dehydrogenase (GDH), 10 mg of glucose, 25 µM of FAD, NAD⁺ with different concentrations, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 8.

**Table 8**

| NAD⁺ (mM) | Conversion rate |
|---|---|
| 3 | - |
| 5 | -+ |
| 10 | -+ |
| 20 | -+ |
| 25 | - |
| 30 | - |

| | |
|---|---|
| Note: in the table above, - indicated a decrease in conversion rate; + indicated an increase in conversion rate; and -+ indicated no change in conversion rate. | |

According to the results in Table 8, next optimization was performed by selecting 5 mM of the NAD⁺.

Reaction condition 8: a 1 mL reaction system included 2 mg of the olefin substrate 1, 50 mg of enzyme powder of styrene monooxygenase StyA, 10 mg of enzyme powder of reductase StyB, 7.5 mg of enzyme powder of Glucose Dehydrogenase (GDH), 10 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate through HPLC. Results were shown in Table 9.

**Table 9**

| Glucose (mg) | Conversion rate |
|---|---|
| 2.5 | - |
| 5 | -+ |
| 7.5 | -+ |
| 10 | -+ |
| 12.5 | - |
| 15 | - |

| | |
|---|---|
| Note: in the table above, - indicated a decrease in conversion rate; + indicated an increase in conversion rate; and -+ indicated no change in conversion rate. | |

According to results in Table 9, it might be learned that the glucose in the reaction system might be reduced to 5 mg.

From the single-factor optimization results of the above reaction conditions, it was seen that an optimal reaction condition at a current phase (in 1 mL system) included the olefin substrate with the concentration being 2 mg/mL, 7.5 mg of the GDH, 25 µM of the FAD, 5 mM of the NAD⁺, and 5 mg of the glucose, with the pH value being 7.5 and reaction temperature being 35 °C.

### Embodiment 3

A catalytic action of the styrene monooxygenase obtained in Embodiment 1 to an olefin substrate 2 was tested. 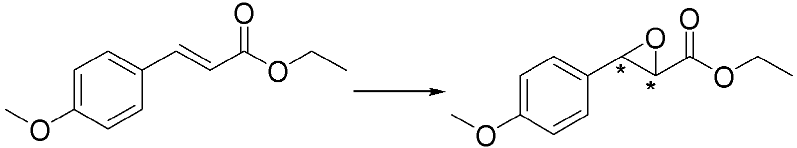 * indicated a chiral carbon atom.

Experimental conditions were as follows: a 1 mL reaction system included 2 mg of the olefin substrate 2, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 10.

**Table 10**

| Oxidase number | Conversion rate |
|---|---|
| 1 | * |
| 2 | * |
| 3 | * |
| 4 | ** |
| 5 | ** |
| 6 | ** |
| 7 | ** |
| 8 | * |
| 9 | ** |
| 10 | * |
| 11 | ** |
| 12 | ** |
| 13 | ** |
| 14 | * |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 4

A catalytic action of the styrene monooxygenase obtained in Embodiment 1 to an olefin substrate 3 was tested.

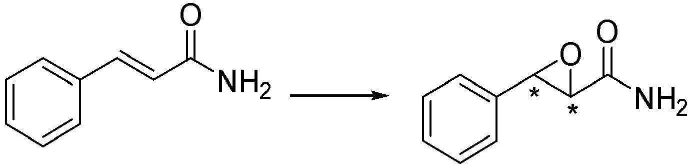

Experimental conditions were as follows: a 1 mL reaction system included 2 mg of the olefin substrate 3, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 11.

**Table 11**

| Oxidase number | Conversion rate |
|---|---|
| 1 | ** |
| 2 | * |
| 3 | * |
| 4 | ** |
| 5 | * |
| 6 | ** |
| 7 | ** |
| 8 | * |
| 9 | ** |
| 10 | * |
| 11 | * |
| 12 | *** |
| 13 | ** |
| 14 | * |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 5

A catalytic action of the styrene monooxygenase obtained in Embodiment 1 to an olefin substrate 4 was tested.

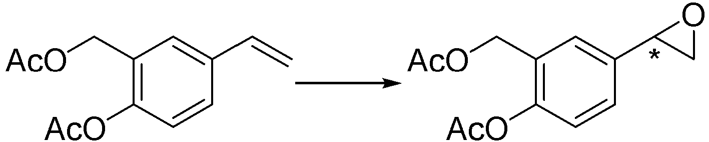

Experimental conditions were as follows: a 1 mL reaction system included 2 mg of the olefin substrate 4, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 12.

**Table 12**

| Oxidase number | Conversion rate |
|---|---|
| 1 | ** |
| 2 | * |
| 3 | * |
| 4 | ** |
| 5 | * |
| 6 | ** |
| 7 | ** |
| 8 | * |
| 9 | ** |
| 10 | * |
| 11 | * |
| 12 | *** |
| 13 | ** |
| 14 | * |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 6

A catalytic action of the styrene monooxygenase obtained in Embodiment 1 to an olefin substrate 5 was tested.

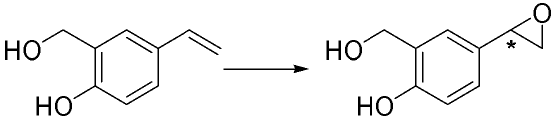

Experimental conditions were as follows: a 1 mL reaction system included 2 mg of the olefin substrate 5, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 13.

**Table 13**

| Oxidase number | Conversion rate |
|---|---|
| 1 | ** |
| 2 | * |
| 3 | * |
| 4 | * |
| 5 | *** |
| 6 | ** |
| 7 | * |
| 8 | * |
| 9 | ** |
| 10 | * |
| 11 | * |
| 12 | *** |
| 13 | ** |
| 14 | * |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 7

A catalytic action of the styrene monooxygenase obtained in Embodiment 1 to an olefin substrate 6 was tested.

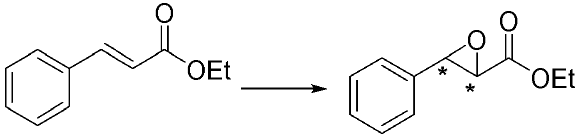

Experimental conditions were as follows: a 1 mL reaction system included 2 mg of the olefin substrate 6, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 14.

**Table 14**

| Oxidase number | Conversion rate |
|---|---|
| 1 | *** |
| 2 | *** |
| 3 | * |
| 4 | * |
| 5 | *** |
| 6 | ** |
| 7 | *** |
| 8 | *** |
| 9 | ** |
| 10 | *** |
| 11 | *** |
| 12 | *** |
| 13 | ** |
| 14 | *** |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 8

A catalytic action of the styrene monooxygenase obtained in Embodiment 1 to an olefin substrate 7 was tested.

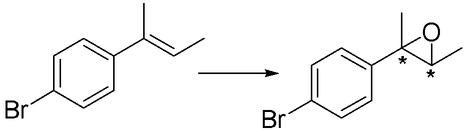

Experimental conditions were as follows: a 1 mL reaction system included 2 mg of the olefin substrate 7, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 15.

**Table 15**

| Oxidase number | Conversion rate |
|---|---|
| 1 | * |
| 2 | * |
| 3 | * |
| 4 | * |
| 5 | * |
| 6 | ** |
| 7 | * |
| 8 | ** |
| 9 | * |
| 10 | ** |
| 11 | * |
| 12 | * |
| 13 | * |
| 14 | * |
| 15 | ** |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 9

A catalytic action of the styrene monooxygenase obtained in Embodiment 1 to an olefin substrate 8 was tested.

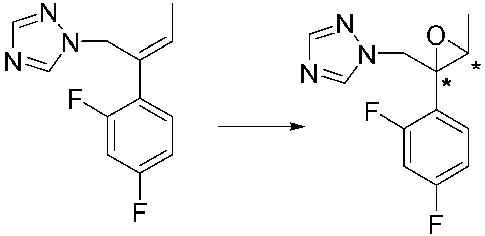

Experimental conditions were as follows: a 1 mL reaction system included 2 mg of the olefin substrate 8, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 16.

**Table 16**

| Oxidase number | Conversion rate |
|---|---|
| 1 | * |
| 2 | *** |
| 3 | * |
| 4 | * |
| 5 | * |
| 6 | * |
| 7 | * |
| 8 | * |
| 9 | * |
| 10 | ** |
| 11 | * |
| 12 | * |
| 13 | ** |
| 14 | ** |
| 15 | * |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 10

A catalytic action of the styrene monooxygenase obtained in Embodiment 1 to an olefin substrate 9 was tested.

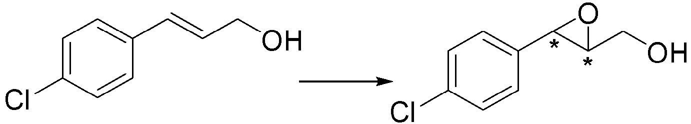

Experimental conditions were as follows: a 1 mL reaction system included 2 mg of the olefin substrate 9, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 17.

**Table 17**

| Oxidase number | Conversion rate |
|---|---|
| 1 | * |
| 2 | ** |
| 3 | * |
| 4 | *** |
| 5 | *** |
| 6 | * |
| 7 | * |
| 8 | *** |
| 9 | * |
| 10 | ** |
| 11 | *** |
| 12 | * |
| 13 | * |
| 14 | ** |
| 15 | * |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 11

A catalytic action of the styrene monooxygenase obtained in Embodiment 1 to an olefin substrate 10 was tested.

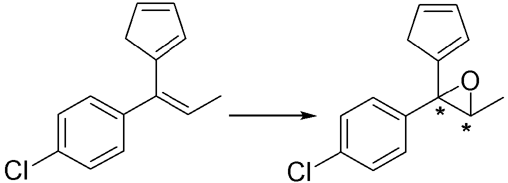

Experimental conditions were as follows: a 1 mL reaction system included 2 mg of the olefin substrate 10, 50 mg of enzyme powder of styrene monooxygenase StyA, 10 mg of enzyme powder of reductase StyB, 7.5 mg of enzyme powder of Glucose Dehydrogenase (GDH), 5 mg of glucose, 25 µM of FAD, 5 mM of NAD⁺, and 100 mM of a phosphate buffer solution with a pH value being 7.5, and the reaction was performed for 16 h at 35 °C. After the reaction ended, 2 mL of acetonitrile was added to the system to terminate the reaction, full shaking and well mixing were performed, then centrifugation was performed for 1 min at 8000 rpm to obtain a supernatant aqueous phase, and the supernatant aqueous phase was sent to detect a conversion rate and chirality through HPLC and GC. Results were shown in Table 18.

**Table 18**

| Oxidase number | Conversion rate |
|---|---|
| 1 | * |
| 2 | ** |
| 3 | * |
| 4 | *** |
| 5 | *** |
| 6 | * |
| 7 | * |
| 8 | *** |
| 9 | * |
| 10 | ** |
| 11 | *** |
| 12 | * |
| 13 | * |
| 14 | ** |
| 15 | * |

| | |
|---|---|
| Note: in the table above, * indicated that the conversion rate was less than 1%; ** indicated that the conversion rate was greater than or equal to 1% and less than 10%; *** indicated that the conversion rate was greater than or equal to 10% and less than 20%; and **** indicated that the conversion rate was greater than or equal to 20% and less than 30%. | |

### Embodiment 12

Product chirality identification was performed on the reaction of No. 2 oxidase and No. 3 oxidase with an olefin substrate 8. Experimental conditions were as follows: a 100 mL reaction system included 200 mg of the olefin substrate, 5000 mg of enzyme powder of styrene monooxygenase StyA, 1000 mg of enzyme powder of reductase StyB, 750 mg of enzyme powder of Glucose Dehydrogenase (GDH), 500 mg of glucose, 2.5 mM of FAD, 500 mM of NAD⁺, and 100 mM of a phosphate buffer solution with a pH value being 7.5, and the reaction was performed for 70 h at 35 °C. After the reaction ended, extraction was performed by using ethyl acetate, column chromatography was performed for purification, white powder was obtained after evaporation drying, the white powder was redissolved with ethanol and sent to determine chirality through GC, and results were shown in Table 19 below.

**Table 19**

| Oxidase number | Product de value |
|---|---|
| 2 | >99% (S, S) |
| 3 | >99% (R, S) |

**It** might be seen from the above description that, in the above embodiments of the present disclosure, the following technical effects were realized.

Although the steric hindrance at both ends of an ethylene double bond in the olefin compound having the above structure is relatively large, the present disclosure uses the above raw material system to perform the enzyme catalysis reaction and use the oxidase therein to obtain the chiral epoxide. Compared to a traditional metal catalyst synthesis method, the method for synthesizing chiral epoxide catalyzed by oxidase has the advantages of a short synthesis route, a wide range of substrate types, high selectivity, mild reaction conditions, less three wastes, and low costs.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure shall all fall within the scope of protection of the present disclosure.

## Claims

1. A method for synthesizing chiral epoxide catalyzed by styrene monooxygenase, wherein the synthesis method comprises:
performing an enzyme catalysis reaction on a raw material system comprising oxidase, an olefin compound, reductase, dehydrogenase, a hydrogen donor, FAD, and NAD⁺, so as to obtain chiral epoxide, wherein
structural formulas of the olefin compound and chiral epoxide respectively are as follows: 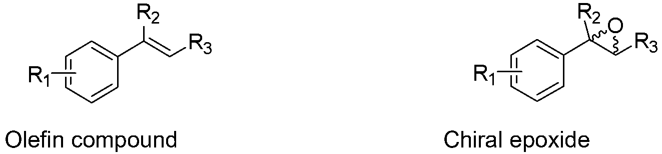
R₂ and R₃ are each independently selected from any one of H, substituted or unsubstituted C₁-C₆ alkyl, substituted or unsubstituted C₁-C₆ heteroalkyl, a substituted or unsubstituted C₂-C₆ ester group, substituted or unsubstituted C₁-C₄ acylamino, substituted or unsubstituted C₃-C₆ cycloalkyl, substituted or unsubstituted C₃-C₆ cycloenyl, or substituted or unsubstituted C₆-C₁₂ aryl; and
R₁ is selected from any one of halogen, hydroxy, carboxyl, acetoxy, substituted or unsubstituted C₁-C₆ alkyl, or substituted or unsubstituted C₁-C₆ heteroalkyl.

2. The synthesis method according to claim 1, wherein a heteroatom on the heteroalkyl is selected from any one or more of N, O, and S; and R₂ and R₃ are each independently selected from any one of H, substituted or unsubstituted C₁-C₄ alkyl, substituted or unsubstituted C₁-C₄ alkoxy, a substituted or unsubstituted C₂-C₄ ester group, substituted or unsubstituted C₁-C₂ acylamino, substituted or unsubstituted C₃-C₅ cycloalkyl, substituted or unsubstituted C₃-C₅ cycloenyl, or substituted or unsubstituted C₆-C₁₀ aryl.

3. The synthesis method according to claim 1 or 2, wherein R₂ and R₃ are each independently selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl; and
when substituents are present on R₂ and R₃, the substituents are selected from any one or more of hydroxy, carboxyl, methyl, ethyl, nitro, methylamino, acetoxy, and five-membered ring heteroaryl or six-membered ring heteroaryl.

4. The synthesis method according to claim 1 or 2, wherein R₂ and R₃ are each independently selected from any one of H, methyl, ethyl, phenyl, 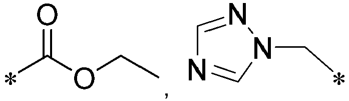 formamido, hydroxymethyl, or cyclopentadienyl.

5. The synthesis method according to claim 1 or 2, wherein the heteroatom on the heteroalkyl is selected from any one or more of N, O, and S; R₁ is selected from any one of halogen, hydroxy, carboxyl, substituted or unsubstituted C₁-C₄ alkyl, or substituted or unsubstituted C₁-C₄ alkoxy; and
when a substituent is present on R₁, the substituent is selected from any one or more of hydroxy, carboxyl, methyl, ethyl, nitro, methylamino, and acetoxy.

6. The synthesis method according to claim 5, wherein R₁ is selected from any one of H, methyl, ethyl, propyl, butyl, methoxy, ethoxy, propoxy, butoxy, hydroxymethyl, an ethyl formate group, an ethyl acetate group, a methyl acetate group, formamido, acetyl, cyclobutyl, cyclopentyl, cyclobutadienyl, cyclopentadienyl, phenyl, or biphenyl.

7. The synthesis method according to claim 5, wherein R₁ is selected from any one of F, Cl, hydroxyl, methyl, ethyl, methoxy, hydroxymethyl, 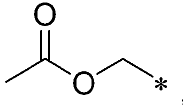 acetoxy.

8. The synthesis method according to claim 1 or 2, wherein the olefin compound is selected from any one or more of 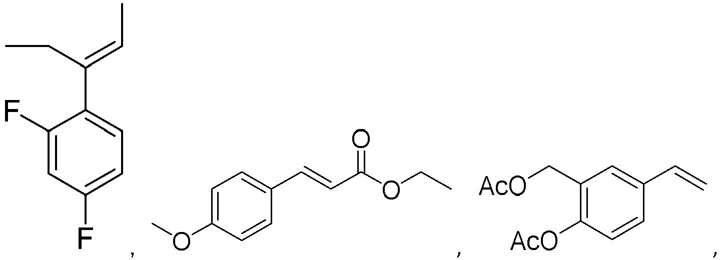 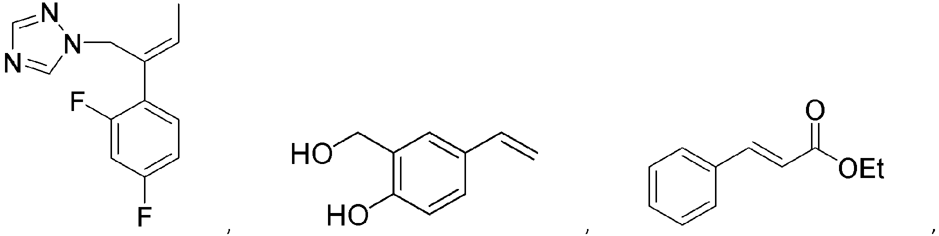 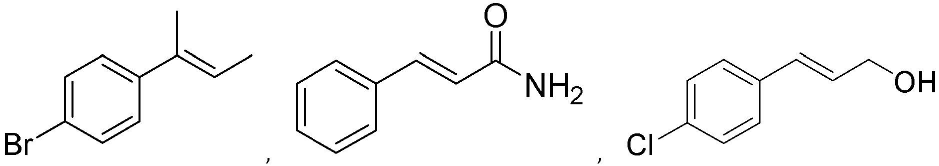 and

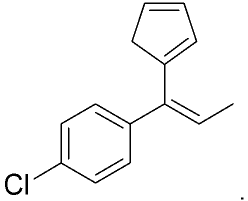

9. The synthesis method according to claim 1 or 2, wherein the oxidase is selected from any one or more of oxidase with sequence being derived from *Marinobacterium litorale* DSM 23545 and NCBI serial number being WP_027855270.1, oxidase with sequence being derived from *Sphingopyxis fribergensis* and NCBI serial number being AJA07151.1, oxidase with sequence being derived from *Streptomyces exfoliatus* and NCBI serial number being WP_137992763.1, oxidase with sequence being derived from *Bradyrhizobium* sp. ORS 375 and NCBI serial number being WP_009031806.1, oxidase with sequence being derived from *Amycolatopsis albispora* and NCBI serial number being WP_113694022.1, oxidase with sequence being derived from *Celeribacter baekdonensis* and NCBI serial number being WP_107722930.1, oxidase with sequence being derived from *Rhodococcus* sp. ST-5 and NCBI serial number being BAL04132.1, oxidase with sequence being derived from *Nocardia farcinica* and NCBI serial number being MBF6535966.1, oxidase with sequence being derived from *Pseudomonas aeruginosa* and NCBI serial number being WP_121207564.1, oxidase with sequence being derived from *Pseudomonas oryzae* and NCBI serial number being WP_090348206.1, oxidase with sequence being derived from *Pseudomonas* sp. SXM-1 and NCBI serial number being WP_134326109.1, oxidase with sequence being derived from *Pseudonocardia* sp. SID8383 and NCBI serial number being WP_161151635.1, oxidase with sequence being derived from *Streptomyces* sp. WAC01280 and NCBI serial number being WP_125743710.1, oxidase with sequence being derived from *Pseudomonas putida* S12 and NCBI serial number being AJA17113.1, and oxidase with sequence being derived from *Variovorax paradoxus* EPS and NCBI serial number being ADU39062.1.

10. The synthesis method according to claim 9, wherein the oxidase is selected from any one or more of the oxidase with the sequence being derived from *Sphingopyxis fribergensis* and NCBI serial number being AJA07151.1, the oxidase with the sequence being derived from *Bradyrhizobium* sp. ORS 375 and NCBI serial number being WP_009031806.1, and the oxidase with the sequence being derived from *Rhodococcus* sp. ST-5 and NCBI serial number being BAL04132.1, the oxidase with the sequence being derived from *Pseudomonas aeruginosa* and NCBI serial number being WP_121207564.1, and the oxidase with the sequence being derived from *Pseudomonas oryzae* and NCBI serial number being WP_090348206.1.

11. The synthesis method according to claim 1 or 2, wherein a temperature for the enzyme catalysis reaction is 20-40 °C, and/or a time for the enzyme catalysis reaction is 16-72 h.

12. The synthesis method according to claim 1 or 2, wherein a mass ratio of the oxidase in the raw material system to the raw material system is 10-50:1; and/or the mass of the oxidase is 20-100 mg; and/or a mass ratio of the reductase to the raw material system is 1-10:1; and/or the mass of the reductase is 2-20 mg; and/or a mass ratio of the hydrogen donor to the raw material system is 1-10:1; a mass ratio of the total mass of the FAD and the NAD⁺ to the raw material system is 10-1000:1; and/or
the reductase is reductase StyB that is able to regenerate FADH₂, and/or the dehydrogenase and the hydrogen donor respectively are Glucose Dehydrogenase (GDH) and glucose; and/or the dehydrogenase and the hydrogen donor respectively are alcohol dehydrogenase and isopropanol; and/or
a mass concentration of the olefin compound is 0.5-2 mg/mL; and/or
a source of the reductase StyB is selected from any one or more of *Pseudomonas* sp. LQ26, *Bradyrhizobium* sp. ORS 375, *Pseudomonas putida, Pseudomonas fluorescens, Pseudomonas* sp. Y2, *Rhodococcus* sp. ST-5, and *Rhodococcus* sp. ST-10; and/or
the raw material system comprises a buffer solution, the buffer solution is selected from any one or more of a phosphate buffer solution, a Tris-HCl buffer solution, and a boric acid buffer solution, and/or a pH value of the buffer solution is 7.5-10.
